# EUROPEAN PATENT SPECIFICATION

(11) **EP 0 539 541 B1**
(45) Date of publication and mention of the grant of the patent: **09.07.1997**
(21) Application number: 92909158.5
(22) Date of filing: 04.05.1992
(51) Int. Cl.: C08L 3/02, C08L 67/04

(54) **BIODEGRADABLE POLYMERIC COMPOSITIONS BASED ON STARCH AND THERMOPLASTIC POLYMERS**
Biologisch abbaubare Polymermassen auf der Basis von Stärke und thermoplastische Polymere
COMPOSITIONS POLYMERES BIODEGRADABLES A BASE D'AMIDON ET DE POLYMERES THERMOPLASTIQUES

(30) Priority: 03.05.1991 IT TO910327; 01.08.1991 EP 91112942; 10.03.1992 IT TO920199; 27.03.1992 IT TO920282
(43) Date of publication of application: 05.05.1993
(73) Proprietor: NOVAMONT S.p.A., 28100 Novara (IT)
(72) Inventor: BASTIOLI, Catia, I-28100 Novara (IT); BELLOTTI, Vittorio, I-28010 Fonataneto d'Agogna (IT); DEL TREDICI, Gianfranco, I-21018 Sesto Calende (IT); LOMBI, Roberto, I-28100 Novara (IT); MONTINO, Alessandro, I-27038 Robbio Lomellina (IT); PONTI, Roberto, I-28047 Oleggio (IT)
(74) Representative: Rambelli, Paolo
(86) International application number: EP9200959
(87) International publication number: WO9219680

(56) References cited:
- EP-A- 0 255 752
- EP-A- 0 400 532
- EP-A- 0 409 782
- EP-A- 0 444 880
- EP-A- 0 575 349
- WORLD PATENTS INDEX LATEST Week 9042, Derwent Publications Ltd., London, GB; AN 90-315322 & JP-A-2 222 421

## Description

The present invention relates to polymeric compositions comprising starch and synthetic thermoplastic polymers, suitable for producing substantially biodegradable articles having satisfactory physical and mechanical properties by conventional techniques for the processing of thermoplastic materials.

Compositions are known from EP-A-0 255 752 comprising starch in a non-destructured form and a polymer of 3-hydroxy-3-alkylpropionic acid. JP-A-2 222 421 describes compositions obtained from an emulsion of a polyhydroxy acetic/polyhydroxyvaleric acid copolymer, which may also contain starch, wherein starch is present in a non-destructured form. EP-A-0 444 880 describes compositions comprising non-destructured starch and a biodegradable polymer.

Thermoplastic compositions of the type mentioned above are known and available on the market and are described for instance in patent applications Nos. EP-A-0 032 802, EP-A-0 327 505, WO90/10671, EP-A-0 400 532, EP-A-0 404 723, EP-A-0 404 727, EP-A-0 404 728, WO91/02024 and WO91/02025.

EP-A-0 575 349, corresponding to WO92/14782, which is prior art only under Art.54(3) EPC, relates to polymeric compositions consisting of starch, ethylene-vinyl alcohol copolymers, polyvinyl alcohol and modified polyvinyl alcohol together with aliphatic polyesters and specific plasticisers which are suitable to avoid the bleeding of the plasticisers out of the composition.

Typically, these compositions are obtainable by blending a starch-based component and a synthetic thermoplastic component under conditions which are typical of extrusion cooking, that is in the presence of a limited amount of water or plasticiser (typically 5-40% by weight referred to the starch-water system), at a temperature and pressure sufficient to destroy the crystallinity of starch and obtain a thermoplastic melt. By means of such process, thermoplastic blends have been obtained, wherein the starch-based component and the synthetic thermoplastic component form a structure which is interpenetrated or at least partially interpenetrated.

Compositions containing destructured starch and a polysaccharide copolymer grafted with a polymerizable monomer are described in EP-A-0 409 782.

An object of the present invention is to provide novel polymeric compositions of the type mentioned above, which have a high biodegradation rate and have improved mechanical properties and/or improved resistance to water and improved low permeability to water vapour in comparison with the corresponding properties of the known starch-based compositions.

A further object of the invention is to improve processability of known biodegradable polymers, thereby to provide novel blends comprising such polymers, which are biodegradable and suitable to be more easily processed into finished articles, particularly sheets, films and filaments.

The above objects are achieved by a polymeric composition obtainable by extruding under temperature and shear conditions to render the polymeric components compatible from the rheological view point a melt comprising a starch component and a synthetic thermoplastic polymer component selected from the group consisting of one of the following polymers or mixtures of polymers:
a) homopolymers of aliphatic hydroxy acids having from 2 to 24 carbon atoms, the corresponding lactones or lactides;
b) copolymers of aliphatic hydroxy acids having from 2 to 24 carbon atoms, the corresponding lactones or lactides with monomers selected from the group consisting of aliphatic hydroxy acids having from 2 to 24 carbon atoms other than that constituting the first monomer, corresponding lactones or lactides, aromatic hydroxy acids, aliphatic or aromatic isocyanates;
c) block or graft copolymers between the homopolymers and copolymers a) or b) with one or more of the following components:
   i) cellulose or modified cellulose such as cellulose acetate, carboxymethylcellulose;
   ii) amylose, amylopectin, natural or modified starches;
   iii) polymers deriving from reaction of diols, polyesters pre-polymers or polymers having diol terminal groups with:
      - aromatic or aliphatic bifunctional isocyanates,
      - aromatic or aliphatic bifunctional epoxides,
      - aliphatic bicarboxylic acids,
      - bicarboxylic cycloaliphatic acids,
      - aromatic acids or anhydrides,
   iv) polyurethanes, polyamide-urethanes from diisocyanates and aminoalcohols, polyamides, polyesters-amides from bicarboxylic acids and aminoalcohols, polyester-urea from amino acids and diesters of glycols,
   v) polyhydroxylated polymers selected from the group consisting of polyvinylalcohol, ethylene-vinylalcohol copolymers, totally or partially hydrolysed and polysaccharides up to destrines;
   vi) polyvinylpyrrolidone, polyvinylpyrrolidone-vinylacetate copolymers, polyethyloxazolines;
   vii) ionomeric polymers selected from polyacrylates and polymetacrylates;
d) polyesters obtained from monomers or comonomers such as defined above at a) and b) upgraded with chain extenders selected from the group consisting of isocyanates, epoxides, phenylesters and aliphatic carbonates;
e) polyesters obtained from monomers and comonomers defined at a) and b) above partially cross-linked by means of polyfunctional acids selected from the group consisting of trimellitic acid, pyromellitic acid, polyisocyanates and polyepoxides, the compositions obtained from the following components being excluded:

- starch, a synthetic polymer selected from ethylene-vinylalcohol copolymers, polyvinylalcohol and modified polyvinylalcohol and mixtures thereof, a second synthetic component selected from a) and b) polymers and a plasticiser other than sorbitol and acetic acids esters of glycerine and selected from the following groups:
   - polyols containing from 1 to 20 repeating hydroxylated units each unit including from 2 to 6 carbon atoms, provided that when the polyol is formed by only one repeating unit it has at least 4 carbon atoms;
   - ethers, thioethers, inorganic and organic esters, acetals and amino-derivatives of polyols, formed by from 1 to 20 repeating hydroxylated units each including from 2 to 6 carbon atoms;
- polyol reaction products having from 1 to 20 repeating hydroxylated units each including from 2 to 6 carbon atoms with chain extenders;
- polyol oxidation products having from 1 to 20 repeating hydroxylated units each including from 2 to 6 carbon atoms including at least one aldehydic or carboxylic functional group or mixtures thereof.

Homopolymers and copolymers of epsilon hydroxyacids are preferred, particularly of 6-hydroxycaproic acid, 6-hydroxyoctanoic, 3,7-dimethyl-6-hydroxyoctanoic acid and corresponding lactones.

As monomers of aliphatic hydroxyacids having from 2 to 24 carbon atoms the following acids and corresponding lactides or lactones are particularly contemplated:
- alpha-hydroxyacids such as lactic acids and the corresponding lactide, glycolic acid and the corresponding glycolide;
- beta-hydroxyacids such as hydroxypropionic acid, hydroxypivalic and hydroxypelargonic acid and the corresponding lactone;
- gamma-hydroxyacids such as hydroxybutyrric and the corresponding lactone;
- delta-hydroxyacids such as hydroxyvaleric acid and the corresponding lactone;
- epsilon-hydroxyacids such as those mentioned above;
- hydroxyacids having the hydroxy group placed beyond the 6-position such as 10-hydroxydecanoic acid; products of natural origin such as sabinic acid (12-hydroxydodecanoic) and juniperic acid (16-hydroxyhexadecanoic); unsaturated hydroxyacids such as ricinoleic; acids deriving from alphahydroxylation of fatty acids such as miristic, palmitic and stearic acids; acids deriving from hydroxylation of unsaturated fatty acids such as oleic, ricinoleic, linolenic and erucic acids;
- cycloaliphatic hydroxyacids such as the hydroxyacids of cyclohexane and of 2,2,2-bicyclooctane.

As copolymers of aliphatic hydroxyacids with isocyanates, copolymers of epsilon-caprolactone with 4,4'-diphenylmethane-diisocyanate (MDI), toluilendiisocyanate (TDI), isophoron diisocyanate or hexanmethylen diisocyanate are preferred.

As the copolymers of aliphatic hydroxyacids and the corresponding lactones with aromatic hydroxyacids copolymers of epsilon-caprolactone with beta-phenyl lactic acid or mandelic acid are preferred.

The crystalline polymers a) - e) which are used within the frame of the present invention have typically a melting point of from 40 to 175°C; however, both crystalline and amorphous polymers may be used; homopolymers and copolymers having a molecular weight above 40,000 are preferred.

It is known that aliphatic polyesters having low melting point or low glass transition temperature are difficult to be processed by conventional techniques for thermoplastic materials, such as film blowing and blow moulding. With reference particularly to poly-epsilon-caprolactone and its copolymers which have low melting point, films produced thereby are tacky, as extruded, and noisy to the touch and have a low melt strength over 130°C; moreover, due to the low crystallization rate of such polymers, the crystallization process proceeds for a long time after production of the finished articles with an undesirable change of properties with time. It has been found that the blending of starch with polymers a) - e) allows to improve their processability properties without impairing the mechanical properties and biodegradability properties thereof; the improvement is particularly effective with polymers having low melting point temperature of from 40 to 100°C.

The relative ratio between the starch component and the polymeric component may vary within wide limits depending upon the final use to which the blends of the invention are adapted. Typically ratios of from 1:9 and 9:1 are used, preferably of from 1:4 to 4:1, more preferably of from 1.5:1 to 1:1.5.

Preferred embodiments comprise blends wherein the polymeric component consists of poly-epsilon-caprolactone, preferably with a molecular weight above 40,000, or of a copolymer of epsilon-caprolactone and aliphatic or aromatic isocyanates, such as the copolymer Estane (registered trademark, BF Goodrich), or mixtures thereof having a weight ratio preferably of from 5:1 to 1:1.

According to another embodiment of the invention, the synthetic polymeric component comprises a component A consisting of polymers cited at items a) through e) or mixtures thereof and a component B comprising one or more polymers or copolymers derived from ethylenically unsaturated monomers having repeating units provided with at least a functional polar group, such as preferably hydroxyl or carboxyl, carboxyalkyl, alkylcarboxyl, pyrrolidyl and acetal, (alkyl is meant to include preferably C1 - C4).

The polymers of component B comprise ethylene copolymers having melting point between 80 and 130°C and an ethylene content higher than about 50 % wt, such as particularly copolymers ethylene-acrylic acid, ethylene-vinylalcohol, ethylene-vinylacetate and mixtures thereof. Particularly preferred are, however higher melting polymers, such as polyvinylalcohol and copolymers of ethylene-vinylalcohol with an ethylene content of from 10 to 44% by weight which are produced by hydrolysis of the corresponding polyvinylacetate and ethylene-vinylacetate, with hydrolysis degree between 50 and 100 %.

Blends of starch and ethylene-vinylalcohol copolymers are described in EP-A-0 400 532 incorporated herein by reference.

The alcohol groups of the polymers mentioned above may be partially or completely modified to produce:
1) ethers resulting from reaction with:
   - ethylene oxide
   - ethylene oxide substituted with alkyl radicals up to C₂₀ or with aromatic radicals
   - acrylonitrile (Ce²⁺ initiator)
   - acrylamide
   - arylalkylhalides
   - chloroacetic acid
   - methylchloromethyl ether
   - silanes
2) inorganic and organic esters such as sulphates, nitrates, phosphates, arsenates, xanthates, carbamates, urethanes, borates, titanates,
3) organic esters resulting from reaction with aliphatic or aromatic acids, chloroacyls, particularly of fatty acids or anhydrides,
4) acetals and ketals produced by reaction with
   - aliphatic aldehydes with up to 22 carbon atoms,
   - unsaturated aliphatic aldehydes with up to 22 carbon atoms,
   - chloroacetaldehyde
   - glyoxal
   - aromatic aldehydes
   - cyclic aliphatic aldehydes,
   - aliphatic ketones
   - arylalkyl ketones
   - alkylcycloalkyl ketones.

The reactions which produce the ethers, organic and inorganic esters and the acetals given above can easily be brought about as described in Chapter 9 and in the literature cited in the publication "Polyvinyl alcohol" edited by C.A. Finch.

It is also possible to use multifunctional polymers of polyvinyl alcohol and of ethylene-vinyl alcohol (containing up to 40% by weight of ethylene with degrees of hydrolysis of the acetate of between 100 and 50%), in which up to 50% of the ethylene may be substituted by co-monomers selected from the group consisting of:
propylene, isobutene, styrene, vinyl chloride, 1,1-dichloroethene, vinyl ethers of the formula CH₂=CR-OR' in which R is hydrogen or a methyl group and R' is an alkyl group with from 1 to 18 carbon atoms, a cycloalkyl group or a polyether, acrylonitrile, methacrylonitrile, vinyl ketones of the formula CH₂=CR-CO-CH₂-R' in which R is hydrogen or a methyl group and R' is hydrogen or a C₁-C₆ alkyl group, acrylic or methacrylic acid and their esters of the formula CH₂=CR-COOR' in which R is hydrogen or a methyl group and R' is hydrogen or a C₁-C₆ alkyl group, and the alkali metal or alkaline-earth metal salts of those acids, vinyl derivatives of the formula CH₂=CR-OCOR' in which R is hydrogen or a methyl group and R' is hydrogen, a methyl group, a methyl group mono-, bi-, or tri-substituted with chloro- or fluoro- groups, or a C₂-C₆ alkyl group, vinyl carbamates of the formula CH₂=CR-CONR'R" in which R is hydrogen or a methyl group and R' and R" are the same or different and are hydrogen or C₁-C₃ alkyl groups, maleic anhydride, fumaric anhydride, vinylpyrrolidone, vinylpyridine, or 1-vinylimidazole.

The copolymerisation is achieved with the use of radical initiators such as hydrogen peroxide, peroxysulphates and benzoyl peroxides, as described in the chapter "Polymerisation processes of vinyl esters" and the literature cited on pages 406 et. seq. of Volume 17 of the "Encyclopedia of Polymer Science and Engineering"

Preferred compositions according to the invention comprise, as component A, poly-epsilon-caprolactone or its copolymers, preferably isocyanate copolymers, polyhydroxybutyrrate, polyhydroxybutyrrate/valerate, or lactic acid polymers or mixtures thereof and, as component B, poly-ethylene-vinyl alcohol, possibly modified as mentioned above, polyvinyl alcohol or poly-ethylene-acrylic acid or poly-ethylene-vinyl-acetate or mixture thereof.

According to a further embodiment, the invention comprises blends wherein the synthetic polymer component comprises, as component A, aliphatic polyesters, including the above mentioned polymers a) through e) and polymers obtained by polymerizations of aliphatic diols with bicarboxylic acids such as polyethylene and polybutylene adipate or sebacate and, as component B, the same polymers mentioned in the preceding paragraph.

Biodegradable polymeric compositions comprising starch and ethylene-vinyl alcohol copolymers are known and provide films having outstanding mechanical properties and fair properties of resistance to water. Such compositions suffer however of some drawbacks particularly relating to a loss of mechanical properties due to humidity changes; particularly below 10°C and low humidity there is an undesirable increase of brittleness and loss of the impact strength which constitutes a limitation in connection with their use in manufacturing articles for packaging and the like applications. On the other hand, as we have mentioned before, polymers a) - e) which generally have good biodegradability properties, are difficult to be processed and due to their low melting point may be used only in a limited range of temperatures.

According to the invention, it has been found that the addition to polymers A of the starch component and of the B polymer component is suitable to improve the mechanical properties and achieve a lower permeability to water vapour and liquid water. Particularly, the addition of starch and polymers B component to polymers A has a nucleating effect, which under the processing conditions of the blends of the invention, provides for a substantial increase of the rate of crystallization of polymers A. The advantage is particularly effective in connection with the production of films, sheets, fibers and filaments and molded articles by film blowing, extrusion, melt spinning, blow molding and injection molding.

Blends including starch, polymers B and plasticizers are available on the market under the trademark Mater Bi by Novamont and such blends may be used as a source for starch and B polymers to provide the blends of the present invention.

The weight ratio between component A and B of the synthetic polymeric component is preferably of from 1:6 to 6:1, more preferably of from 1:4 to 4:1.

Preferred composition specifically adapted for injection molding comprise:
- from about 20 to about 60 % wt starch component,
- from about 10 to about 80 % wt, particularly from about 10 to about 50 % wt component A,
- from 0 to about 45 % wt component B, particularly from about 2 to about 30 % wt component B,
the percent amounts being expressed with reference to the sum of starch and total synthetic component.

With reference to compositions adapted for the production of films, sheets and the like, the preferred compositions comprise:
- from about 5 to about 60 % wt starch component,
- from about 40 to about 80 % wt component A,
- from 0 to about 35 % wt, particularly from about 5 to about 30 % wt component B,
the percent amounts being expressed with reference to the sum of the starch and total synthetic component.

When the available product Mater Bi (Novamont trademark) is used as the source for starch and type B polymers, preferred weight ratios between type A polymer and Mater Bi are from 80:20 to 30:70.

The starch used in the polymeric compositions is preferably a native starch extracted from vegetables such as potatoes, rice, tapioca, maize and cereals. In any case, the term starch is intended also to include physically and chemically modified starches.

It has been found that the use of a starch component comprising at least 78% wt amylopectin allows to obtain films by extrusion blowing, the cross section of which, when observed by scanning electromicroscopy (X 2,000 magnification), shows a layered or lamellar structure made by a plurality of linear polymeric microphases alternated with starch micro phases in which the crystalline structure of starch is no longer identifiable. The achievement of such layered structure allows to substantially increase the barrier properties against gases and liquids of films and sheets obtained from such polymeric blends.

In this respect a starch component having an amylopectin content higher than 90 % wt and more desirably higher than 94 % wt is preferred. A starch component complying with the above requirements may be obtained by using waxy starch (amylopectin content of about 95 % wt) as the sole starchy material or mixtures of amylopectin , waxy starch and/or conventional starch with lower amylopectin content such as mais starch and potato starch.

On the other hand a starch component having an amylopectin content lower than about 78% wt and correspondingly an amylose content higher than about 22% wt appears to favour under the processing conditions of extrusion and film blowing, an interprenetration of the starch component with the synthetic polymer component.

The above mentioned layered or lamellar structure may also be obtained by use of a starch component having a lower amylopectin content, higher than about 70% wt, by adding to the blend being extruded any substance which is capable of reducing the complexing power of amylose or capable of interacting with starch by means of hydrophilic interactions such as boric acid, borax, methaboric acid, aluminium hydroxide and alkali metals salts, particularly chlorides. For that purpose an effective amount of such substances is of from 0.01 to 10% wt, preferabiy of from 0.05 to 5% wt referred to the starch component.

The addition of boron containing compounds in order to improve transparency of starch / polymer blends is described in U.S. Serial No. 734492 filed July 23, 1991, incorporated herein by reference.

The polymeric composition preferably includes a plasticizer at a concentration of from 1 to 50% by weight, preferably from 5 to 40%, more preferably from 5 to 25% wt, with reference to the weight of the total composition consisting of the starch and polymeric component(s). Polyols selected from the following are used as the plasticizer in the composition wherein the synthetic thermoplastic component is selected from the polymers and copolymers listed in a) to e) :
a) polyols formed by from 1 to 20 repeating hydroxylated units each including from 2 to 6 carbon atoms,
b) ethers, thioethers, inorganic and organic esters, acetals and amino-derivatives of polyols formed by from 1 to 20 repeating hydroxylated units each including from 2 to 6 carbon atoms,
c) reaction products of polyols having from 1 to 20 repeating hydroxylated units each including from 2 to 6 carbon atoms with chain extenders,
d) oxidation products of polyols having from 1 to 20 repeating hydroxylated units each including from 2 to 6 carbon atoms including at least one aldehydic or carboxylic functional group or a mixture thereof.

Compounds which have vapour pressures below that of glycerine at ambient temperature (25°C) and which are soluble in water are preferred, in order to reduce bleeding or sweating phenomena of the plasticizer from finished articles obtained from the compositions of the invention.

The aliphatic polyols of type a) include compounds of the formula:

OH-CH₂-(CHOH)ₙ-CH₂OH (I)

in which n is from 0 to 4, such as ethylene glycol, glycerol, erythritol, arabitol, adonitol, xylitol, mannitol, iditol, galactitol, sorbitol and allitol and polyols which do not fall within the formula given above, such as propylene glycol, polyglycols, trimethylolpropane, pentaerythritol, polyvinyl alcohol with from 3 to 20 repeating units, and polyglycerol formed by from 2 to 10, preferably from 2 to 5, monomeric units, including mixtures of various oligomers.

The aliphatic polyol derivatives of paragraph b) preferably have structural formulae which can be obtained by the substitution of at least one alcoholic functional group of the polyol in question, which is preferably selected from those cited in the preceding paragraph, by a functional group selected from:
-O-(CH₂)ₙ-H in which n = 1-18, preferably 1-4,
-O-CH-CH-R₁ in which R₁ = H or -CH₃,
-O(CH₂-CHR₁-O)ₙ-H in which R₁ = H or CH₃ and n = 1-20,
-O-(CH₂)ₙ-Ar in which Ar is a simple, substituted, or heterocyclic aromatic radical and n = 0-4,
-OCO-H,
-OCO-CR₁R₂R₃ in which the R₁, R₂, and R₃ groups are the same or different and are selected from H, Cl, and F,
-OCO-(CH₂)ₙ-H in which n = 2-18, preferably 2-5,
-ONO₂,
-OPO₃M₂ in which M may be H, ammonium, an alkali metal, an alkaline earth, or an organic cation, particularly trimethylammonium, pyridinium or picoline,
-SO₃-Ar in which Ar is benzene or toluene,
-OCO-CH(SO₃M)-COOM in which the Ms are the same or different and are selected from H, an alkali metal, an alkaline-earth metal, ammonium, and an organic cation, particularly pyridinium, picoline or methylammonium,
-OCO-B-COOM in which B is (CH₂)ₙ in which n = 1-6 or -CH=CH-, M may be H, an alkali metal, an alkaline-earth metal, or -(CH₂)ₙH in which n = 1-6 or an aryl group,
-OCONH-R₁ in which R₁ may be -H or an aliphatic or aromatic radical,
-O-(CH₂)ₙ-COOM in which n = 1-6 and M may be H, an alkali metal, an alkaline-earth metal, ammonium, or an organic cation, particularly pyridinium, trimethylammonium, or picoline,
-O-(CH₂)ₙ-COOR₁ in which n = 1-6, R₁ = H(CH₂)ₘ- in which m = 1-6,
-NR₁R₂ in which R₁ and R₂ = H, CH₃-, CH₃CH₂-, -CH₂-CH₂OH or a salified amino group,
-O-(CH₂)ₙ-NR₁R₂ in which n = 1-4, R₁ and R₂ = H, CH₃-, CH₃CH₂-, or CH₂-CH₂OH, and in which the amino group may be salified, 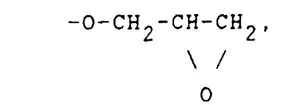
-O-CH₂-CHOH-CH₂-NR₁R₂ in which R₁ and R₂ are the same or different and are selected from H, and H(CH₂)ₙ in which n = 1-6 and in which the amino group may be salified,
-O-CH₂-CHOH-CH₂-R⁺₁Cl⁻ in which R⁺₁ is a trialkylammonium, pyridinium or picoline group,
-O-(CH₂)ₙR⁺₁Cl⁻ in which n = 1-6 and R⁺₁ is a trialkylammonium, pyridinium or picoline group,
-O-(CH₂)ₙ-CN in which n = 1-6,
-O-(CH₂)ₙ-CONH₂ in which n = 1-6,
-O-(CH₂)ₘ-SO₂-(CH₂)ₙ-H in which m and n = 1-4,
-SCSNH₂,
-O-SiX₃ and -O-SiOX₃ in which X may be an aliphatic or an aromatic radical.

Mono- and di-ethers and mono- and di-esters of the polyols of formula (I) given above are particularly preferred and monoethoxylate, monopropoxylate, and monoacetate derivatives, particularly of sorbitol, are most preferred.

The compounds of paragraph c) result from the joining of two or more polyol molecules by means of chain extenders, in particular such as bicarboxylic acids, aldehydes and isocyanates.

Preferred compounds are of the formula:

R-CH₂-(CHR)ₙ-CH₂-O-A-O-CH₂-(CHR)ₘ-CH₂-R

in which n and m are the same or different and have values of from 1 to 6, the R groups are the same or different and are hydroxyl groups or have the meaning given above, and in which A is selected from the group consisting of:
-CHR₁ in which R₁ = H or H-(CH₂)ₙ-, in which n = 1-5 (acetals),
-(CH₂)ₙ- in which n = 1-6,
-(CH₂-O-CH₂)ₙ in which n = 1-20,
-(CH₂CH₂-O)ₙ-CH₂CH₂- in which n = 1-20,
-OC-(CH₂)ₙ-CO- in which n = 0-6,
-OC-Ar-CO- in which Ar is an aromatic radical which is also heterocyclic,
-PO₂-,
-CONH-(CH₂)ₙNHCO-,
and compounds of the formula:

R-CH₂-(CHR)ₙ-CH₂-A-CH₂-(CHR)ₘ-CH₂-R

in which n and m are the same or different and are whole numbers from 1 to 6, the R groups are the same or different and are hydroxyl groups or have the meaning given above, and in which A is selected from the group consisting of NH- and -NH-(CH₂-CH₂-NH)ₙ- in which n is a whole number from 1 to 6.

Of the compounds given above, compounds in which only one of the R groups is a group forming an ether or an ester are preferred.

The term "polyol" is intended to include mono- and polysaccharides having up to 20 monosaccharide units.

The following monosaccharides are considered in particular:
- pentoses and their derivatives of the formula: 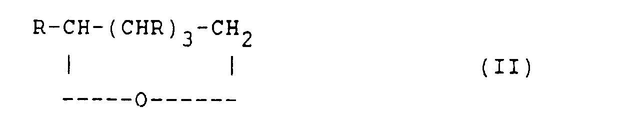 in which the R groups are the same or different and are hydroxyl groups or have the meaning given above.
   Examples of these compounds are arabinose, lyxose, ribose and xylose and, preferably, their monoethers and monoesters,
- aldohexoses and their derivatives of the formula: 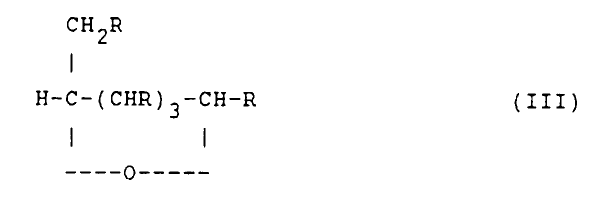 and ketohexoses and their derivatives of the formula: 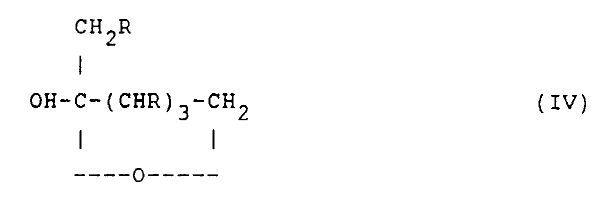 in which the R groups are the same or different and are hydroxyl groups or have the meaning given above.

Examples of these monosaccharides are glucose, fructose, mannose, allose, altrose, galactose, gulose, idose, inositol, sorbose and talitol.

Of their etherified or esterified derivatives, monoethoxylate and monopropoxylate derivatives and monoesters, particularly of acetic acid, are preferred.

The polysaccharides include compounds having up to 20 repeating units of formula (II), (III) or (IV) with molecular weights up to that of dextrin.

The R functional groups may be introduced into the basic structure of the polyol by known reactions, for example, as described in Chapter 9 and in the literature cited in the publication "Polyvinyl alcohol" edited by C.A. Finch.

Preferred plasticizers comprise: glycerine, polyglycerol glycerol ethoxylate, ethylene or propylene glycol,ethylene or propylene diglycol, ethylene or propylene triglycol, polyethylene or polypropylene glycol, 1,2-propandiol, 1,3-propandiol,1,3-, 1,4-butandiol, 1,5-pentandiol, 1,5-hexandiol, 1,2,6-, 1,3,5-hexantriol, neopentylglycol, trimethylolpropane, pentaerythritol, sorbitol acetate, sorbitol diacetate, sorbitol monoethoxylate, sorbitol dipropoxylate, sorbitol diethoxylate, sorbitol hexaethoxylate, aminosorbitol, trihydroxymethy laminomethane, glucose/PEG, the product of reaction of ethylene oxide with glucose, trimethylolpropane monoethoxylate, mannitol monoacetate, mannitol monoethoxylate, butyl glucoside, glucose monoethoxylate, alpha-methyl glucoside ,the sodium salt of carboxymethylsorbitol, polyglycerol monoethoxylate and mixtures thereof.

The plasticizer used with compositions comprising blends of A) and B) polymers is selected from the groups consisting of glycerine, glycerine mono and diacetate, ethylene and propylene glycols, ethylene and propylene diglycols, ethylene and propylene triglycols, polyethylene and polypropylene glycols, 1,2- and 1,3- propondiols, sorbitol.

If synthetic polymers with high melting points such as, for example, polyvinyl alcohol and ethylene-vinyl alcohol copolymer with an ethylene content no greater than 44% by weight are used as component B, the decribed plasticizer may also perform an important function in the process which leads to the formation of a composition with an (at least partially) interpenetrated structure. The melting points of these polymers (160-200°C) are so high that complete interpenetration with the starch molecules is not possible; the addition of plasticizers common to the starchy and polymeric components lowers the melting points of the synthetic polymers and, at the same time, changes their rheological behaviour.

The polymeric material may also include agents, such as urea or hydroxides of alkali metals or of alkaline-earth metals, which can destroy hydrogen bonds and of which quantities of between 0.5 and 20% with reference to the weight of the entire composition are added to the mixture of starch and copolymer.

The addition of urea, in the preferred amount of from 5 to 20% wt, is advantageous particularly for the production of blends for film-blowing.

The composition of the invention may also comprise relatively low amounts of hydrophobic polymers ,such as polyethylene, polypropylene and polystyrene; however in order not to impair the biodegradability properties, the amount of such polymers should preferably not exceed about 5% wt of the overall composition.

The polymeric composition of the invention may also include cross-linking agents such as aldehydes, ketones and glyoxals, process coadjuvants and release agents, and lubricants which are normally incorporated in compositions for moulding or extrusion, such as fatty acids, esters of fatty acids, higher alcohols, polythene waxes, LDPE, fungicides, flame-proofing agents, herbicides, antioxidants, fertilisers, opacifiers and stabilisers.

Further additives comprise polyvinylpyrrolidone, polyoxazoline, cellulose acetates and nitrates, regenerated cellulose, alkyl cellulose, carboxymethyl cellulose, casein-type proteins and salts thereof, natural gums such as gum arabic, algin and alginates, chitin and chitosan.

The polymeric compositions are preferably prepared by the mixing of the components cited above in an extruder heated to a maximum temperature generally between 100 and 220°C by the methods described in patent applications EP-A-413798 and EP-A-400532 incorporated herein by reference. The composition supplied to the extruder includes water due to the intrinsic water content of the starch used (9-15% by weight) and water may be added as appropriate.

The composition of the invention have preferably a water content of from 1.5 to 5% wt ,as extruded ,before conditioning, which is obtained by reducing the initial water content of the total composition during the extrusion by intermediate de-gassing.

The pressures to which the mixture is subjected during the thermal processing treatment are typical for extrusion in single or double-screw extruders. Although the process is preferably carried out in an extruder, the starch, synthetic polymer and plasticizer may be mixed by any device which ensures conditions of temperature and shearing stress suitable to make the starch and the polymer used compatible from a rheological point of view.

The method for preparing the compositions of the invention, under the preferred conditions, includes the following steps:
- swelling the starch component and the synthetic polymer component by means of the plasticizer, and possibly water, at a temperature of between 80 and 180°C with a dynamic change in their melting points and rheological behaviour; this effect can be achieved, for example, during a first stage of the transportation of the components through an extruder, for periods of the order of from 2 to 50 seconds,
- subjecting the mixture to shearing conditions corresponding to similar viscosity values of the plasticized starch and synthetic polymer components,
- de-gassing freely or in a controlled manner under pressure or under vacuum to produce a melt at a temperature of 140-180°C with a liquid content such that bubbles are not created at atmospheric pressure, that is, for example, at the output of the extruder,
- cooling the finished product in a water bath or in air.

The entire method requires a pressure of between 0.5 and 10 MPa, preferably between 1 and 5 MPa.

As stated, the thermoplastic composition is preferably prepared by mixing the components mentioned directly, but the starch may also be treated beforehand in the presence of plasticizers, and possibly added water, with temperature conditions of from 100 to 220°C to produce a thermoplastic starch. This starch may be mixed with the synthetic polymer and a further quantity of plasticizer during a second step. For polyvinyl alcohol and ethylene-vinyl alcohol copolymer, a portion of the total quantity of plasticizer is added at the start of the mixing of the pretreated starch and the synthetic polymer since the plasticizer has to be available to modify the melting point and rheological behaviour of the polymer to make it compatible with the starch.

The blends of the invention, may be obtained by direct blending, preferably by means of a conventional extruder, of starch and polymeric components A and B, when the latter is used or, according to the latter embodiment, by blending the component A polymer a) to e) with previously obtained blends obtained from starch and B polymers.

### Example 1

38 parts of mais starch Globe 03401 (Cerestar, registered trademark, intrinsic water content 12% wt), 38 parts of ethylene-vinylalcohol copolymer with an ethylene molar content of 44% and hydrolysis degree of the acetate groups of 99.5%, 5 parts urea, 0.3 parts Armide E, 12 parts sorbitol mono-ethoxylated, 3.7 parts glycerine and 3 parts water were fed to a twin screw extruder OMC of 58 mm, L/D equal to 36, provided with 9 heating zones with a degassing section. The following operative conditions were selected for the extrusion process:
Screw speed (rpm): 170
degassing pressure (bar): 0.9
position of the degassing section: 8th zone thermal profile: cold zone / 90°C / 140°C / 180°C / 180°C / 180°C / 180°C / 175°C / 165°C
head temperature: 145°C with melt temperature: 145°C
head pressure (bar): 27
absorption (A): 67-69

The extrudate in spaghetti-like form was cooled in a water bath and transformed into pellets; the pellets had an intrinsic water content of 3.5% by weight (product named in the following pellet Al).

The following composition:
70 parts of poly-epsilon-caprolactone (PCL "TONE P-787", registered trademark Union Carbide)
30 parts of the pellets Al
4 parts glycerine
was fed to an OMC extruder,diameter 20mm, compression ratio 1:3, having four controlled temperature zones, without intermediate degassing section, operating under the following conditions:
screw speed: 45 rpm
thermal profile : 140/145/150/155°C

The extrudate in spaghetti-like form was transformed into pellets (pellets A2). The pellets A2 were fed to a HAAKE extruder with a diameter of 19 mm and L/D equal to 25, operating at a speed of 45 rpm with the thermal profile: 140°C / 145°C / 150°C / 155°C, provided with a blowing head, and transformed into films having a thickness between 30 and 50 micron. The mechanical properties of the obtained films are given in Table 1.

### Example 2

The procedure of example 1 was repeated by using in formulating the pellets A2 the following composition:
70 parts PCL "TONE P-787" (Union Carbide)
30 parts of the pellets Al
6 parts glycerine

The mechanical and permeability properties are given in Table 1.

### Example 3

The procedure of example 1 was repeated by using in formulating the pellets A2 the following composition:
60 parts PCL "TONE P-787" (Union Carbide),
40 parts of the pellets A1

The mechanical properties are given in Table 1.

### Example 4

The procedure of example 1 was repeated by using in formulating the pellets A2 the following composition:
50 parts PCL "TONE P-787" (Union Carbide),
50 parts of the pellets Al

The mechanical and permeability properties are given in Table 1.

### Example 5

50 parts of the pellets Al and 50 parts of poly-epsilon-caprolactone P-787 (Union Carbide) were fed to same OMC extruder used for the production of pellets Al without intermediate degassing section operating under the following conditions:
Screw speed: 130 rpm
thermal profile: first cold zone / 90°C / 130°C / 150°C / 155°C / 160°C / 160°C / 160°C / 150°C
head temperature: 145°C
pressure: 32 bar
absorption: 105 A
productivity: 60 kg/h

The thus obtained pellets were fed to a blowing apparatus with a diameter of 44 mm and L/D equal to 30, provided with a film blowing head having the following features:
die diameter: 100 mm
die gap: 0.5 mm
die land: 10

The conditions for film blowing were the following:
Screw speed: 65 rpm
thermal profile of the extruder: 135°C / 140°C / 140°C / 140°C
neck thermal profile: 140°C
head thermal profile: 135°C
draw ratio: 3.1
blow-up ratio: 3.2

Films having an average thickness of 50 microns were obtained. The tensile properties and tear strength properties and permeability of the thus obtained films are given in Table 2.

### Example 6

The procedure of example 5 was repeated by feeding to the film blowing device a composition comprising 47.5% wt of the pellets A1, 50% wt of poly-epsilon-caprolactone and 2.5% wt polyethylene. The features of the thus obtained films are given in Table 2.

### Example 7

The procedure of example 5 was repeated by feeding to the film blowing device a composition comprising 47.5% wt of the pellets Al, 50% wt of poly-epsilon-caprolactone and 2.5% wt urea. The features of the films are given in Table 2.

### Example 8

The procedure of example 5 was repeated by feeding to the film blowing device pellets obtained from 48.5% wt of the pellets Al, 48.5% wt of poly-epsilon-caprolactone, 2.4% wt water and 0.6% wt glycerine. The features of the thus obtained films are given in Table 2.

### Examples 9 - 16

The procedures of examples 1 - 8 were repeated by using, instead of poly-epsilon-caprolactone P-787 (Union Carbide) alone, a blend 4:1 wt of the latter and of a copolymer derived from epsilon-caprolactone and bifunctional isocyanate (polyester/urethane) produced by BF Goodrich (Estane, registered trademark); the properties of the thus obtained films were comparable to those of the films obtained according to examples 1 - 8.

### Examples 17 - 23

To a twin screw extruder APV having a diameter of 30 mm and L/D equal to 25, the composition given in the following Table 3 were directly fed. In some cases, which are indicated by an asterix in Table 3, the poly-epsilon-caprolactone polymer was pre-blended with ethylene-vinylalcohol copolymer (ethylene molar content 44%, hydrolysis degree 99.5%) by means of an extruder. The APV extruder was operated according to the following conditions:
Screw speed: 170 rpm
productivity: 13.1 kg/h
thermal profile: first cold zone / 60°C / 160°C / 165°C / 170°C / 170°C / 170°C / 170°C / 160°C / 150°C
head temperature: 140°C

The extrudate in spaghetti-like form was pelletized and then fed to an injection moulding press Sandretto to produce dumb-bell specimen (ASTM 638) which were subjected to tests in order to determine their mechanical properties. The mechanical properties are given in Table 3.

### Example 24 - 25: Blow-molding

The following compositions were directly processed by blow-molding to produce plastic bottles:

| | | Ex 24 | Ex 25 |
|---|---|---|---|
| Starch (*) | % wt | 44.6 | 44.6 |
| PCL P-787 (**) | % wt | 41.1 | - |
| Estane(***) | % wt | - | 41.1 |
| Glycerine | % wt | 11.8 | 11.8 |
| Water (added) | % wt | 2.5 | 2.5 |

| | | | |
|---|---|---|---|
| * Starch Globe 03401 (Cerestar , water content 12 % wt ) | | | |
| ** Union Carbide | | | |
| *** BF Goodrich | | | |

The above compositions were fed to a AEMME blow-molding apparatus having a constant taper screw (compression ratio 1:3) with a diameter of 30 mm, L/D equal to 25 with the following operative conditions:
mold type: round bottle ,diameter 50 mm
cooling system: water 17°C
air pressure: 6-7 bar

| | | | Ex 24 | Ex 25 |
|---|---|---|---|---|
| cylinder | T1 | °C | 100 | 150 |
| | T2 | °C | 100 | 150 |
| | T3 | °C | 105 | 150 |
| | | | | |
| head | T4 | °C | 110 | 150 |
| | T5 | °C | 115 | 150 |
| | | | | |
| screw speed: | | rpm | 42 | 42 |
| parison speed | | m/min | 0.7 | 0.7 |
| melt T (T3) | | °C | 106 | 147 |
| blowing time | | sec | 11 | 10 |
| bottle weight | | g | 22.9 | 21.8 |
| wall thickness | | mm | 0.75 | 0.75 |

No relevant difficulties were found in the production of the bottles by blow-molding under the above described conditions. Comparative tests carried out under the same operative conditions with the use of poly-epsilon-caprolactone P-787 alone gave rise to difficulties such as the need to substantially increase the blowing time and problems of tackiness.

### Example 26 - 27

The procedure of Example 1 for the production of pellets Al was repeated with the use of a waxy starch having an amylopectin content of about 95% wt and intrinsic water content equal to about 12% wt (Snowflake 04201 starch, registered trademark).

The thus obtained pellets were blended with poly-epsilon caprolactone P 787 (Union Carbide) directly in the film blowing apparatus Ghioldi under the following conditions with a weight ratio (pellets / poly-caprolactone) 50/50 ( Ex 26) and 60/40 (Ex 27) by weight.
Ghioldi apparatus: diameter 40mm and L/D 30
screw: constant taper, compression ratio 1:2.8
shape: spiral
die diameter: 100 mm
die gap: 0.5 mm
die land: 10
Film blowing conditions:
screw speed: 65 rpm
thermal profile of the extruder: 135/135/140/140°C (melt temperature: 152°C)
neck thermal profile: 140-140°C (melt temperature 152°C)
head thermal profile: 135-135°C (melt temperature 140°C)
neck pressure : 274 bar die pressure: 73 bar
draw ratio: 3
blow-up ratio: 3

The mechanical and permeability properties to water vapour (determined by the method of Lissy 39°C, R.H. 90%) and to liquid water (20°C, part not in contact with water at R.H. below 10%) of the thus obtained films were the following:

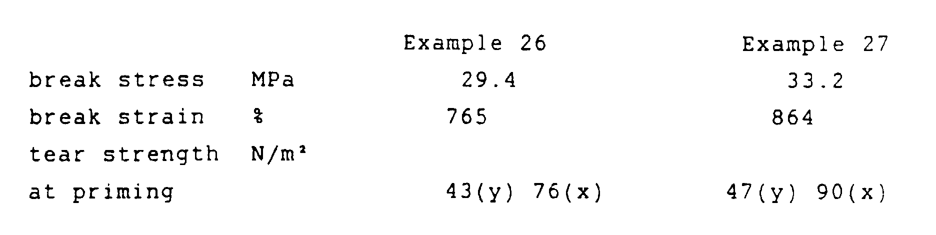

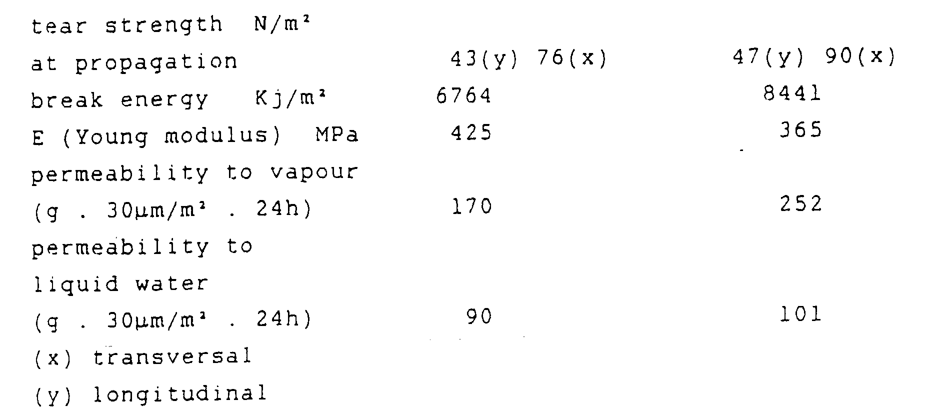

Biodegradability tests carried out on the products obtained from the above example showed a substantial improvement in respect of the known conpositions. Particularly compositions comprising starch, poly-ethylene-vinyl alcohol and poly-epsilon-caprolactone under composting conditions have achieved a biodegradability up to 80 % after only 10 days.

Shaped articles such as films, sheets, laminated films and sheets, filaments, fibers and injection molded articles produced from the herein described compositions by injection molding, extrusion, blow molding, extrusion blowing, thermoforming and similar conventional methods for the processing of thermoplastic matrials fall within the scope of the invention.

Specific applications comprise films for absorbent articles, such as diapers and the like, for mulch and packaging in general, films for protective coatings or films coextruded with biodegradable and non-biodegradable polymers.

Laminated films are described in WO92/02363.

Compositions according to the invention comprising type A and type B polymers are useful to produce selective membranes for pervaporation processes.

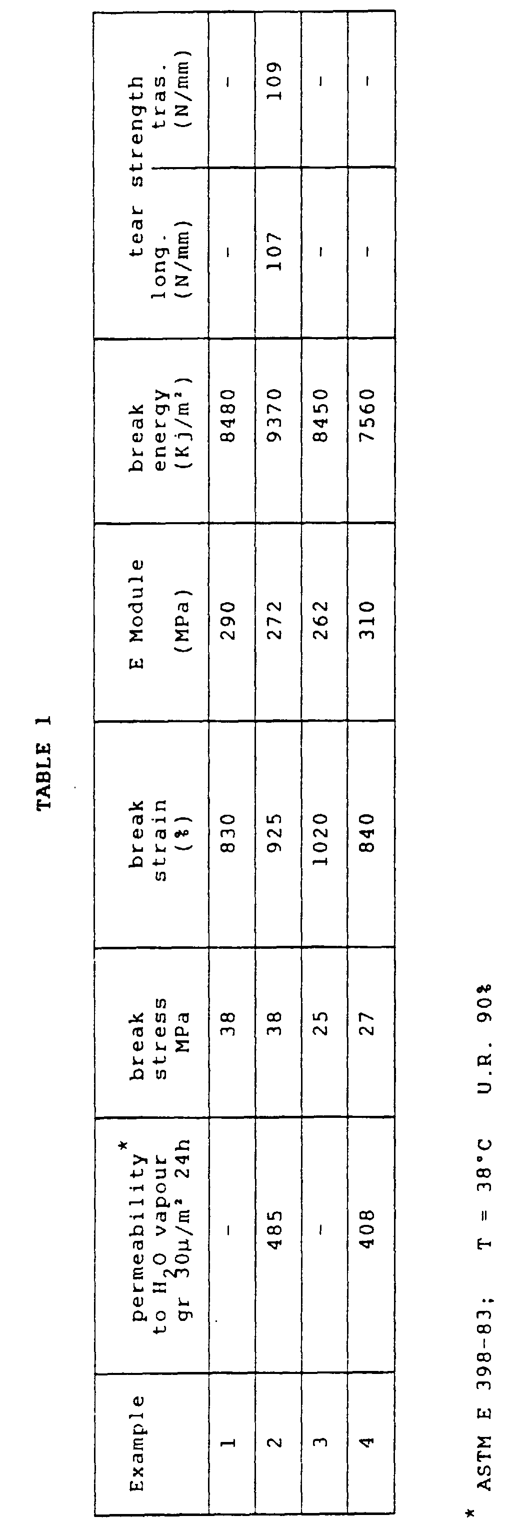

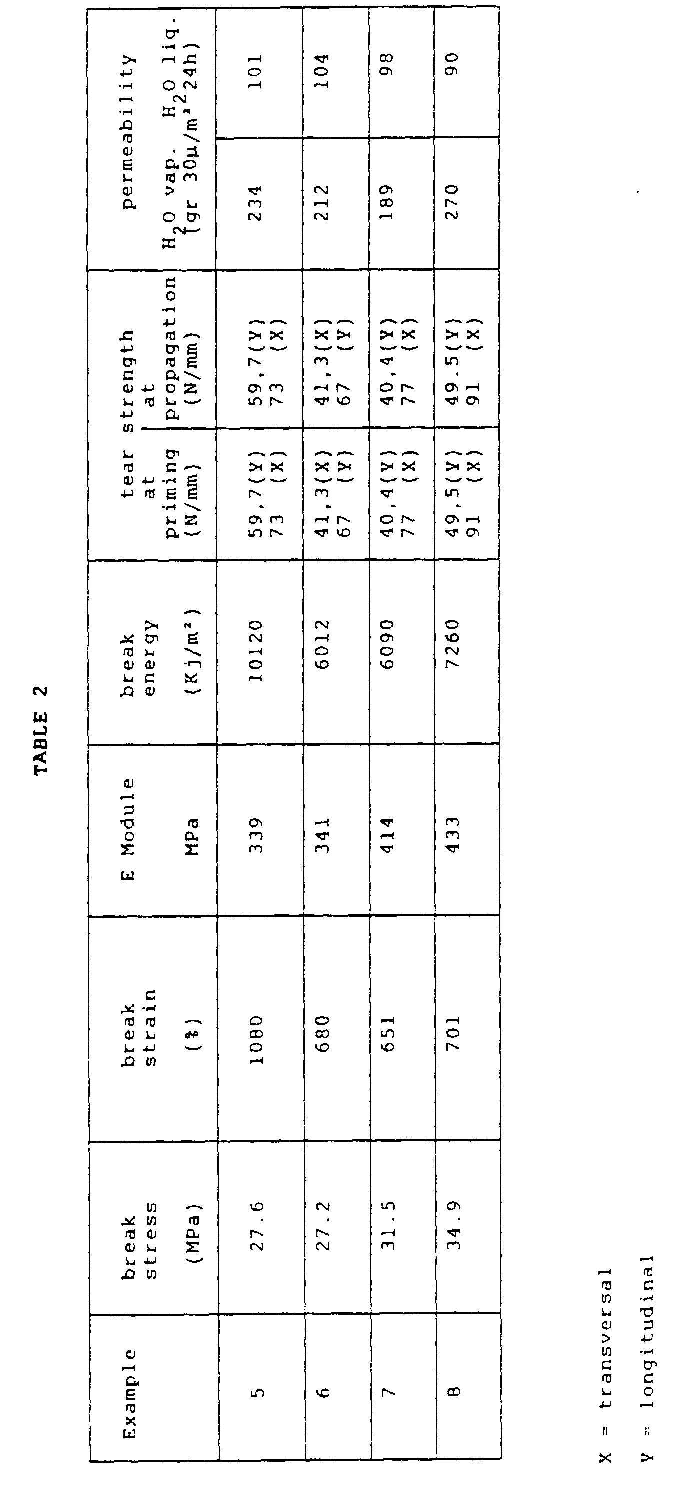

**TABLE 3**

| Example | 17 | 18* | 19* | 20* | 21* | 22 | 23 |
|---|---|---|---|---|---|---|---|
| Starch | 44.6 | 41.2 | 41.2 | 41.2 | 41.2 | 33 | 49 |
| EVOH | - | 32.9 | 24.66 | 32.9 | 24.66 | 32.9 | 20 |
| EAA | - | 3.1 | 3.1 | 3.1 | 3.1 | 2.5 | - |
| Armide E | - | 0.3 | 0.3 | 0.3 | 0.3 | 0.2 | 0.3 |
| Glycerine | 11.8 | 11.8 | 11.8 | 11.8 | 11.8 | 9.4 | 11.8 |
| H₂O | 2.5 | 2.5 | 2.5 | 2.5 | 2.5 | 2 | 2.5 |
| PCL based polymer | 41.1(a) | 8.2(b) | 16.44(b) | 8.2(c) | 16.44(c) | 20(b) | 16.4(b |
| Torque (%) | 56 | 63 | 63ö68 | 64ö68 | 70 | 74ö76 | 82-85 |
| Pressure (bar) | 21-31 | 18 | 18ö23 | 17ö18 | 21 | 17ö20 | 25-27 |
| Final H₂O (% wt) | 3.8 | 3.97 | 3.46 | 3.23 | 3.26 | 2.12 | 3.7 |
| MFR(g/10') | 1.59 | 1.1 | 0.6 | 1.25 | 0.4 | 0.34 | - |
| σ_{b} (MPa) | 9 | 20 | 15.6 | 19.7 | 16 | 23.2 | 17 |
| ε_{b} (%) | 529 | 277 | 221 | 312.4 | 302 | 422 | 131 |
| E (MPa) | 344 | 996 | 646 | 888 | 579 | 949 | 1158 |
| Er (Kj/m²) | 2382 | 2385 | 1485 | 2595 | 2003 | 4062 | 1048 |
| Starch: GLOBE 03401 (Cerestar), as such, instrinsic water content 12% wt | | | | | | | |
| EVOH: poly-ethylene-vinyl alcohol, 44% ethylene molar content, hydrolysis degree 99.5 | | | | | | | |
| EAA : poly-ethylene - acrylic acid, Pow 5981, 20% wt acrylic acid | | | | | | | |
| σ_{b}: break stress | | | | | | | |
| ε_{b}: break strain | | | | | | | |
| (a:) poly-epsilon-caprolactone P-787 (Union Carbide) | | | | | | | |
| (b): ε-caprolactone/diisocyanate copolymer Estane PCL 54353 BF Goodrich | | | | | | | |
| (c): ε-caprolactone/diisocyanate copolymer Estane PCL 54351 BF Goodrich | | | | | | | |
| Es.18*: preblending 80% EVOH + 20% PCL 54353 | | | | | | | |
| Es.19*: preblending 60% EVOH + 40% PCL 54353 | | | | | | | |
| Es.20*: preblending 80% EVOH + 20% PCL 54351 | | | | | | | |
| Es.21*: preblending 60% EVOH + 40% PCL 54351 | | | | | | | |
| MFR: melt flow rate (g/10 min) at 170°C, 5 kg load | | | | | | | |

## Claims

1. A polymeric composition obtainable by extruding under temperature and shear conditions to render the polymeric components compatible from the rheological view point a melt comprising a starch component and a synthetic thermoplastic polymer component selected from the group consisting of one of the following polymers or mixtures of polymers:
a) homopolymers of aliphatic hydroxy acids having from 2 to 24 carbon atoms, the corresponding lactones or lactides;
b) copolymers of aliphatic hydroxy acids having from 2 to 24 carbon atoms, the corresponding lactones or lactides with monomers selected from the group consisting of aliphatic hydroxy acids having from 2 to 24 carbon atoms other than that constituting the first monomer, corresponding lactones or lactides, aromatic hydroxy acids, aliphatic or aromatic isocyanates;
c) block or graft copolymers between the homopolymers and copolymers a) or b) with one or more of the following components:
i) cellulose or modified cellulose such as cellulose acetate, carboxymethylcellulose;
ii) amylose, amylopectin, natural or modified starches;
iii) polymers deriving from reaction of diols, polyesters pre-polymers or polymers having diol terminal groups with:
- aromatic or aliphatic bifunctional isocyanates,
- aromatic or aliphatic bifunctional epoxides,
- aliphatic bicarboxylic acids,
- bicarboxylic cycloaliphatic acids,
- aromatic acids or anhydrides,
iv) polyurethanes, polyamide-urethanes from diisocyanates and aminoalcohols, polyamides, polyesters-amides from bicarboxylic acids and aminoalcohols, polyester-urea from amino acids and diesters of glycols,
v) polyhydroxylated polymers selected from the group consisting of polyvinylalcohol, ethylene-vinylalcohol copolymers, totally or partially hydrolysed and polysaccharides up to destrines;
vi) polyvinylpyrrolidone, polyvinylpyrrolidone-vinylacetate copolymers, polyethyloxazolines;
vii) ionomeric polymers selected from polyacrylates and polymetacrylates;
d) polyesters obtained from monomers or comonomers such as defined above at a) and b) upgraded with chain extenders selected from the group consisting of isocyanates, epoxides, phenylesters and aliphatic carbonates;
e) polyesters obtained from monomers and comonomers defined at a) and b) above partially cross-linked by means of polyfunctional acids selected from the group consisting of trimellitic acid, pyromellitic acid, polyisocyanates and polyepoxides, the compositions obtained from the following components being excluded:
- starch, a synthetic polymer selected from ethylene-vinylalcohol copolymers, polyvinylalcohol and modified polyvinylalcohol and mixtures thereof, a second synthetic component selected from a) and b) polymers and a plasticiser other than sorbitol and acetic acids esters of glycerine and selected from the following groups:
- polyols containing from 1 to 20 repeating hydroxylated units each unit including from 2 to 6 carbon atoms, provided that when the polyol is formed by only one repeating unit it has at least 4 carbon atoms;
- ethers, thioethers, inorganic and organic esters, acetals and amino-derivatives of polyols, formed by from 1 to 20 repeating hydroxylated units each including from 2 to 6 carbon atoms;
- polyol reaction products having from 1 to 20 repeating hydroxylated units each including from 2 to 6 carbon atoms with chain extenders;
- polyol oxidation products having from 1 to 20 repeating hydroxylated units each including from 2 to 6 carbon atoms including at least one aldehydic or carboxylic functional group or mixtures thereof.

2. A composition according to claim 1, wherein the starch-based component and the synthetic polymeric component are in a weight ratio of from 1:9 to 9:1, preferably from 1:4 to 4:1.

3. A composition according to claim 1 and 2, wherein the synthetic component is a mixture of a polymer a) with a copolymer b).

4. A composition according to claim 1, wherein synthetic component is a polymer or a copolymer of an ε-hydroxy acid or a corresponding lactone selected from the group consisting of 6-hydroxycaproic, 6-hydroxyoctanoic and 3,7-dimethyl-6-hydroxyoctanoic acid.

5. A composition according to claim 4, wherein the synthetic component is a copolymer of an ε-hydroxy acid and an isocyanate selected from the group consisting of 4,4'-diphenylmethane-diisocyanate, toluilendiisocyanate, isophorondiisocyanate and hexamethylendiisocyanate.

6. A composition according to claims 1 to 5, wherein the synthetic component is poly-ε-caprolactone or a copolymer of poly-ε-caprolactone and an aliphatic or aromatic isocyanate or a mixture thereof or a copolymer of poly-ε-caprolactone with cellulose, modified cellulose, cellulose acetate or carboxymethylcellulose.

7. A composition according to claim 6, wherein the synthetic component is poly-ε-caprolactone having a molecular weight above 40,000.

8. A composition according to claim 7, wherein the synthetic component is a mixture of poly-ε-caprolactone and copolymer of ε-caprolactone with an aliphatic or aromatic isocyanate in a weight ratio of from 5:1 to 1:1.

9. A composition according to claim 1, wherein the polymeric synthetic component comprises a mixture of a component A selected from polymers a) to e) and mixture thereof and a component B comprising a polymer deriving from ethylenically unsaturated monomers, having repeating units provided with at least a functional polar group selected from the group consisting of hydroxyl, carboxyl, carboxyalkyl, alkylcarboxyl, pyrrolidyl and acetal, said composition comprising a plasticiser selected from the groups consisting of glycerine, glycerine mono- and diacetates, sorbitol, ethylene and propylene glycols, ethylene or propylene diglycols, ethylene or propylene triglycols, polyethylene glycol, polypropylene glycol, 1,2-propandiol, 1,3-propandiol.

10. A composition according to claim 9, wherein component B comprises a polymer selected from the group consisting of polyvinylalcohol, ethylene-acrylic acid, ethylene-vinylacetate, ethylene-vinylalcohol copolymers, modified ethylene-vinylalcohol, modified polyvinylalcohol and mixtures thereof.

11. A composition according to claim 10, wherein component B comprises a polyethylene-vinylalcohol obtained by hydrolysis of the corresponding polyethylene-vinylacetate, having an ethylene content lower than 44% by weight and hydrolysis degree of the acetate groups of from 50 to 100%.

12. A composition according to claim 9, wherein components A and B are present in a weight ratio of from 1:6 and 6:1, preferably of from 1:4 to 4:1.

13. A composition according to claim 9, comprising:
- from about 20 to about 60% wt of the starch component,
- from about 10 to about 80% wt of component A,
- from 0 to about 45% wt of component B,
the percent amounts being expressed with reference to the sum of the starch and total synthetic component.

14. A composition according to claim 13, comprising from about 10 to about 50% wt of component A and from about 2 to about 30% wt of component B.

15. A composition according to claim 9, comprising:
- from about 5 to about 60% wt of the starch component,
- from about 40 to about 80% wt of component A,
- from 0 to about 35% wt of component B,
the percent amounts being expressed with reference to the sum of the starch and total synthetic component.

16. A composition according to claim 15, comprising from about 5 to about 30% wt of component B.

17. A composition according to claims 1 to 8, comprising from 1 to 50% by weight, referred to the starch/synthetic polymer system of a plasticiser selected from the group consisting of glycerine, polyglycerol, glycerol ethoxylate, ethylene or propylene glycol, ethylene or propylene diglycol, ethylene or propylene triglycol, polyethylene or polypropylene glycol, 1,2-propandiol, 1,3-propandiol, 1,2-, 1,3-, 1,4-butandiol, 1,5-pentadiol, 1,5-hexandiol, 1,2,6-, 1,3,5-hexantriol, neopentylglycol, trimethylolpropane, pentaerythritol, sorbitol acetate, sorbitol diacetate, sorbitol monoethoxylate, sorbitol dipropoxylate, sorbitol diethoxylate, sorbitol hexaethoxylate, aminosorbitol, trihydroxymethylaminomethane, glucose/PEG, the product of reaction of ethylene oxide with glucose, trimethylolpropane monoethoxylate, mannitol monoacetate, mannitol monoethoxylate, butyl glucoside, glucose monoethoxylate, α-methyl glucoside, the sodium salt of carboxymethylsorbitol, polyglycerol monoethoxylate and mixtures thereof.

18. A composition according to claim 17, wherein the plasticiser is present in the amount of from 1 to 50% by weight, with reference to the weight of the total composition consisting of the starch and polymeric component(s).

19. A composition according to claim 16, wherein the plasticiser is selected from glycerine and sorbitol ethoxylate and mixtures thereof.

20. A composition according to claim 16, comprising urea in the amount of from 0.5 to 20% wt with reference to the weight of the total composition.

21. A composition according to claims 1 or 8, comprising up to 5% wt with reference to the overall composition of hydrophobic polymer selected from the group consisting of polyethylene, polypropylene and polystyrene.

22. A composition according to claims 1 to 8, wherein the synthetic component comprises a polymer or copolymer as defined in a) to e) of claim 1 and a plasticiser selected from the group as defined in claim 9.

23. A composition according to claim 22, wherein the plasticiser is used in an amount from 1 to 50% on the weight of the composition.

24. A composition according to claims 1 or 8, having a water content of from 1.5 to 5% wt with reference to the overall composition.

25. A composition according to claims 1 to 24, comprising an additive selected from the group consisting of cellulose acetate and nitrate, regenerated cellulose, alkyl cellulose, carboxymethylcellulose.

26. A composition according to claims 1 or 8, wherein the starch component comprises more than 78% wt of amylopectin.

27. A composition according to claims 1 or 8, comprising from 0.01 to 10% wt with reference to the weight of the starch component of an additive selected from the group consisting of boric acid, borax, metaboric acid, aluminium hydroxide and alkali-metal salts.

28. A composition according to claim 23, wherein the starch component has an amylopectin content higher than 70% by weight.

29. A composition according to claim 9, wherein component A comprises a polymer obtained by polymerisation of an aliphatic diol with a bicarboxylic acid.

30. A composition according to claim 29, wherein the polymer obtained by polymerisation of an aliphatic diol with bicarboxylic acid is selected from polyethylene and polybutylene adipates and sebacates.

31. Articles, particularly films, sheets, fibers and filaments as obtainable by the use of a polymeric composition according to claims 1 to 8.

32. A film or a sheet prepared from a composition prepared from a melt comprising starch, polycaprolactone and a plasticiser selected from glycerine and sorbitol or mixtures thereof.

## Patentansprüche

1. Polymer-Zusammensetzung, erhältlich durch Extrudieren einer Schmelze unter Temperatur- und Scherbedingungen, um die polymeren Komponenten aus rheologischer Sicht verträglich zu machen, wobei die Schmelze eine Stärke-Komponente und eine synthetische thermoplastische Polymer-Komponente aufweist, wobei die synthetische thermoplastische Polymer-komponente mindestens ein Polymer oder ein Gemisch von Polymeren ist, die ausgewählt werden aus der Gruppe bestehend aus:
a) Homopolymeren von aliphatischen Hydroxysäuren mit 2 bis 24 Kohlenstoffatomen, den entsprechenden Lactonen oder Lactiden;
b) Copolymeren von aliphatischen Hydroxysäuren mit 2 bis 24 Kohlenstoffatomen, den entsprechenden Lactonen oder Lactiden, mit Monomeren, ausgewählt aus der Gruppe bestehend aus aliphatischen Hydroxysäuren mit 2 bis 24 Kohlenstoffatomen, die verschieden sind von jenen, die das erste Monomer bilden, entsprechenden Lactonen oder Lactiden; aromatischen Hydroxysäuren; aliphatischen oder aromatischen Isocyanaten;
c) Block- oder Pfropf-Copolymeren zwischen den Homopolymeren und den Copolymeren a) oder b) mit einer oder mehreren der folgenden Komponenten:
i) Cellulose oder modifizierte Cellulose, wie z.B. Celluloseacetat, Carboxymethylcellulose;
ii) Amylose, Amylopectin, natürliche oder modifizierte Stärken;
iii) Polymere, stammend aus der Umsetzung von Diolen, Polyester-Vorpolymeren oder Polymeren mit Diol-Endgruppen mit:
- aromatischen oder aliphatischen bifunktionellen Isocyanaten,
- aromatischen oder aliphatischen bifunktionellen Epoxiden,
- aliphatischen Dicarbonsäuren,
- cycloaliphatischen Dicarbonsäuren,
- aromatischen Säuren oder Säureanhydriden,
iv) Polyurethane, Polyamidurethane aus Diisocyanaten und Aminoalkoholen, Polyamide, Polyesteramide aus Dicarbonsäuren und Aminoalkoholen, Polyester-Harnstoff aus Aminosäuren und Diestern von Glycolen,
v) polyhydroxylierte Polymere. ausgewählt aus der Gruppe bestehend aus Polyvinylalkohol, Ethylen-Vinylalkohol-Copolymeren, die vollständig oder teilweise hydrolysiert sind, und Polysacchariden bis zu Destrinen:
vi) Polyvinylpyrrolidon, Polyvinylpyrrolidon-Vinylacetat-Copolymere, Polyethyloxazoline;
vii) ionomere Polymere, ausgewählt unter Polyacrylaten und Polymetacrylaten;
d) Polyestern, erhalten aus Monomeren oder Comonomeren, wie z.B. oben unter a) und b) definiert, die mit Kettenverlängerern, ausgewählt aus der Gruppe bestehend aus Isocyanaten, Epoxiden, Phenylestern und aliphatischen Carbonaten verlängert sind;
e) Polyestern, erhalten aus Monomeren und Comonomeren, wie oben unter a) und b) definiert, teilweise vernetzt mittels polyfunktioneller Säuren, ausgewählt aus der Gruppe bestehend aus Trimellithsäure, Pyromellithsäure, Polyisocyanaten und Polyepoxiden, wobei Zusammensetzungen, die ausgehend von den folgenden Komponenten erhalten werden, ausgeschlossen sind:
- Stärke, ein synthetisches Polymer, ausgewählt unter Ethylen-Vinylalkohol-Copolymeren, Polyvinylalkohol und modifiziertem Polyvinylalkohol und Gemischen davon, eine zweite synthetische Komponente, ausgewählt unter als a) und b) definierten Polymeren, und einem Weichmacher, der verschieden ist von Sorbit und Essigsäureestern des Glycerins, und unter den folgenden Gruppen ausgewählt wird:
- Polyolen mit 1 bis 20 wiederkehrenden hydroxylierten Einheiten, wobei jede Einheit 2 bis 6 Kohlenstoffatome einschließt, mit der Maßgabe, daß, sofern das Polyol aus lediglich einer wiederkehrenden Einheit gebildet wird, diese mindestens 4 Kohlenstoffatome besitzt;
- Ethern, Thioethern, anorganischen und organischen Estern, Acetalen und Amin-Derivaten von Polyolen, die aus 1 bis 20 wiederkehrenden hydroxylierten Einheiten gebildet werden, wobei jede Einheit 2 bis 6 Kohlenstoffatome aufweist;
- Polyol-Umsetzungsprodukten mit Ketienverlängerern, wobei die Polyole 1 bis 20 wiederkehrende hydroxylierte Einheiten mit jeweils 2 bis 6 Kohlenstoffatomen aufweisen;
- Polyol-Oxidationsprodukten mit 1 bis 20 wiederkehrenden hydroxylierten Einheiten, die jeweils 2 bis 6 Kohlenstoffatome und mindestens eine Aldehyd- oder Carbonsäure-funktionelle Gruppe oder Gemische davon aufweisen.

2. Zusammensetzung nach Anspruch 1, wobei die Komponente auf Stärkebasis und die synthetische Polymer-Komponente in einem Gewichtsverhältnis von 1:9 bis 9:1, vorzugsweise von 1:4 bis 4:1, vorliegen.

3. Zusammensetzung nach Anspruch 1 und 2, wobei die synthetische Komponente ein Gemisch eines Polymers a) mit einem Copolymer b) ist.

4. Zusammensetzung nach Anspruch 1, wobei die synthetische Komponente ein Polymer oder ein Copolymer einer ε-Hydroxysäure oder eines entsprechenden Lactons, ausgewählt aus der Gruppe bestehend aus 6-Hydroxycapron-, 6-Hydroxyoctan- und 3,7-Dimethyl-6-hydroxyoctansäure, ist.

5. Zusammensetzung nach Anspruch 4, wobei die synthetische Komponente ein Copolymer einer ε-Hydroxysäure und eines Isocyanats, ausgewählt aus der Gruppe bestehend aus 4,4'-Diphenylmethandiisocyanat, Toluylidendiisocyanat, Isophorondiisocyanat und Hexamethylendiisocyanat, ist.

6. Zusammensetzung nach Ansprüchen 1 bis 5. wobei die synthetische Komponente Poly-ε-Caprolacion oder ein Copolymer von Poly-ε-Caprolacton und einem aliphatischen oder aromatischen Isocyanat oder einem Gemisch davon, oder ein Copolymer von Poly-ε-Caprolacton mit Cellulose, modifizierter Cellulose, Celluloseacetat oder Carboxymethylcellulose ist.

7. Zusammensetzung nach Anspruch 6, wobei die synthetische Komponente Poly-ε-Caprolacton mit einem Molekulargewicht von über 40.000 ist.

8. Zusammensetzung nach Anspruch 7, wobei die synthetische Komponente ein Gemisch aus Poly-ε-Caprolacton und einem Copolymer von ε-Caprolacton mit einem aliphatischen oder aromatischen Isocyanat in einem Gewichtsverhältnis von 5:1 bis 1:1 ist.

9. Zusammensetzung nach Anspruch 1, wobei die polymere synthetische Komponente ein Gemisch einer Komponente A, ausgewählt unter Polymeren a) bis e) und Gemischen davon, und einer Komponente B ist, die ein Polymer aufweist, das abgeleitet wird von ethylenisch ungesättigten Monomeren, wobei das Polymer wiederkehrende Einheiten aufweist, die mit mindestens einer funktionellen polaren Gruppe versehen sind, die ausgewählt wird aus der Gruppe bestehend aus Hydroxyl, Carboxyl, Carboxyalkyl, Alkylcarboxyl, Pyrrolidyl und Acetal, wobei die Zusammensetzung einen Weichmacher aufweist, der ausgewählt wird aus der Gruppe bestehend aus Glycerin, Mono- und Diacetaten des Glycerins, Sorbit, Ethylen und Propylenglycolen. Ethylen- oder Propylendiglycolen, Ethylen- oder Propylentriglycolen, Polyethylenglycol, Polypropylenglycol, 1,2-Propandiol, 1,3-Propandiol.

10. Zusammensetzung nach Anspruch 9. wobei die Komponente B ein Polymer. ausgewählt aus der Gruppe bestehend aus Polyvinylalkohol, Ethylen-Acrylsäure-, Ethylen-Vinylacetat-, Ethylen-Vinylalkohol-Copolymeren, modifiziertem Ethylen-Vinylalkohol, modifiziertem Polyvinylalkohol und Gemischen davon, ist.

11. Zusammensetzung nach Anspruch 10, wobei die Komponente B einen Polyethylen-Vinylalkohol aufweist, der erhalten wird durch Hydrolyse des korrespondierenden Polyethylen-Vinylacetats und einen Ethylengehalt von weniger als 44 Gew.-% sowie einen Hydrolysegrad der Acetatgruppen von 50 bis 100% aufweist.

12. Zusammensetzung nach Anspruch 9, wobei die Komponenten A und B in einem Gewichtsverhältnis von 1:6 bis 6:1, vorzugsweise 1:4 bis 4:1, vorliegen.

13. Zusammensetzung nach Anspruch 9, die umfaßt:
- ungefähr 20 bis ungefähr 60 Gew.-% der Stärke-Komponente,
- ungefähr 10 bis ungefähr 80 Gew.-% der Komponente A,
- 0 bis ungefähr 45 Gew.-% der Komponente B,
wobei die prozentualen Mengen bezogen auf die Summe aus Stärke- und der gesamten synthetischen Komponente ausgedrückt sind.

14. Zusammensetzung nach Anspruch 13, die ungefähr 10 bis ungefähr 50 Gew.-% der Komponente A und etwa 2 bis etwa 30 Gew.-% der Komponente B aufweist.

15. Zusammensetzung nach Anspruch 9, die umfaßt:
- ungefähr 5 bis ungefähr 60 Gew.-% der Stärke-Komponente,
- ungefähr 40 bis ungefähr 80 Gew.-% der Komponente A,
- 0 bis ungefähr 35 Gew.-% der Komponente B,
wobei die prozentualen Mengen bezogen auf die Summe aus Stärke und der gesamten synthetischen Komponente ausgedrückt sind.

16. Zusammensetzung nach Anspruch 15, die ungefähr 5 bis ungefähr 30 Gew.-% der Komponente B aufweist.

17. Zusammensetzung nach Ansprüchen 1 bis 8, die 1 bis 50 Gew.-%, bezogen auf das Stärke/synthetische Polymer-System, eines Weichmachers, der ausgewählt wird aus der Gruppe bestehend aus Glycerin, Polyglycerol, Glycerolethoxylat, Ethylen- oder Propylenglycol, Ethylen- oder Propylendiglycol, Ethylen- oder Propylentriglycol, Polyethylen- oder Polypropylenglycol, 1,2-Propandiol, 1,3-Propandiol, 1,2-, 1,3-, 1,4- Butandiol, 1,5-Pentandiol, 1,5-Hexandiol, 1,2,6-, 1,3,5-Hexantriol, Neopentylglycol, Trimethylolpropan, Pentaerythrit, Sorbitacetat, Sorbitdiacetat, Sorbitmonoethoxylat, Sorbitdipropoxylat, Sorbitdiethoxylat, Sorbithexaethoxylat. Aminosorbit, Trihydroxymethylaminomethan, Glucose/ PEG, dem Produkt der Umsetzung von Ethylenoxid mit Glucose, Trimethylolpropanmonoethoxylat, Mannitmonoacetat, Mannitmonoethoxylat Butylglucosid, Glucosemonoethoxylat, α-Methylglucosid, dem Natriumsalz von Carboxymethylsorbit, Polyglycerolmonoethoxylat und Gemischen davon, umfaßt.

18. Zusammensetzung nach Anspruch 17, wobei der Weichmacher in einer Menge von 1 bis 50 Gew.-%, bezogen auf das Gewicht der gesamten Zusammensetzung, bestehend aus Stärke und polymerer (polymeren) Komponente(n), vorliegt.

19. Zusammensetzung nach Anspruch 16. wobei der Weichmacher ausgewählt wird unter Glycerin und Sorbitethoxylat und Gemischen davon.

20. Zusammensetzung nach Anspruch 16, die Harnstoff in einer Menge von 0,5 bis 20 Gew.%, bezogen auf das Gewicht der gesamten Zusammensetzung, aufweist.

21. Zusammensetzung nach Anspruch 1 oder 8, die bis zu 5 Gew.-%, bezogen auf die Gesamt-Zusammensetzung, eines hydrophoben Polymers, ausgewählt aus der Gruppe bestehend aus Polyethylen, Polypropylen und Polystyrol, aufweist.

22. Zusammensetzung nach Ansprüchen 1 bis 8, wobei die synthetische Komponente ein Polymer oder Copolymer, wie unter a) bis e) gemäß Anspruch 1 definiert, und einen Weichmacher, ausgewählt aus der in Anspruch 9 definierten Gruppe, aufweist.

23. Zusammensetzung nach Anspruch 22, wobei der Weichmacher in einer Menge von 1 bis 50%, bezogen auf das Gewicht der Zusammensetzung, verwendet wird.

24. Zusammensetzung gemäß Anspruch 1 oder 8 mit einem Wassergehalt von 1,5 bis 5 Gew.-%, bezogen auf die Gesamt-Zusammensetzung.

25. Zusammensetzung nach Ansprüchen 1 bis 24, die ein Additiv enthält, das ausgewählt wird aus der Gruppe bestehend aus Celluloseacetat und Cellulosenitrat, regenerierter Cellulose, Alkylcellulose, Carboxymethylcellulose.

26. Zusammensetzung nach Anspruch 1 oder 8. wobei die Stärke-Komponente mehr als 78 Gew.-% Amylopectin aufweist.

27. Zusammensetzung nach Anspruch 1 oder 8, die 0,01 bis 10 Gew.-%, bezogen auf das Gewicht der Stärke-Komponente, eines Additivs enthält, das ausgewählt wird aus der Gruppe bestehend aus Borsäure, Borax, Metaborsäure, Aluminiumhydroxid und Alkalimetall-Salzen.

28. Zusammensetzung nach Anspruch 23, wobei die Stärke-Komponente einen Amylopectin-Gehalt von mehr als 70 Gew.-% aufweist.

29. Zusammensetzung nach Anspruch 9, wobei die Komponente A ein Polymer umfaßt, das durch Polymerisation eines aliphatischen Diols mit einer Dicarbonsäure erhalten wird.

30. Zusammensetzung nach Anspruch 29, wobei das durch Polymerisation eines aliphatischen Diols mit einer Dicarbonsäure erhaltene Polymer ausgewählt wird unter Polyethylen und Polybutylenadipaten und -sebacaten.

31. Gegenstände, insbesondere Folien. Blätter, Fasern und Filamente, erhältlich durch Verwendung einer Polymer-Zusammensetzung nach Ansprüchen 1 bis 8.

32. Folie oder Blatt, hergestellt aus einer Zusammensetzung, die ausgehend von einer Schmelze umfassend Starke, Polycaprolacton und einen Weichmacher, der ausgewählt wird unter Glycerin und Sorbit oder Gemischen davon, hergestellt wird.

## Revendications

1. Une composition polymère susceptible d'être obtenue par extrusion, dans des conditions de températures et de cisaillement rendant les composants polymères compatibles d'un point de vue rhéologique, d'une masse fondue comprenant un composant à base d'amidon et un composant à base de polymère thermoplastique de synthèse, dans laquelle le composant de synthèse est choisi dans le groupe consistant en les suivants polymère ou mélange de polymères:
a) homopolymères d'acides hydroxylés aliphatiques comportant de 2 à 24 atomes de carbone, des lactones ou des lactones correspondants;
b) copolymères d'acides hydroxylés aliphatiques comportant 2 à 24 atomes de carbone, des lactones ou des lactides correspondants, avec des monomères sélectionnés dans le groupe consistant en acides hydroxylés aliphatiques comportant de 2 à 24 atomes de carbone autres que celui constituant le premier monomère, lactones ou lactides correspondants, acides hydroxylés aromatiques, isocyanates aliphatiques ou aromatiques ;
c) copolymères séquencés ou greffés entre les homopolymères et copolyrnères a) ou b) avec un ou plusieurs des composants suivants :
i) cellulose ou cellulose modifiée telle que acétate de cellulose, carboxyméthylcellulose ;
ii) amylose, amylopectine, amidon modifié ou naturel ;
iii) polymères dérivant de la réaction de diols, prépolymères ou polymères de polyester comportant des groupes terminaux diols avec :
- des isocyanates bifonctionnels aromatiques ou aliphatiques,
- des époxydes bifonctionnels aliphatiques ou aromatiques,
- des acides dicarboxyliques aliphatiques,
- des acides dicarboxyliques cycloaliphatiques,
- des acides ou anhydrides aromatiques,
iv) polyuréthanes, polyamide-uréthanes provenant de diisocyanates et d'aminoalcools, polyamides, polyester-amides provenant d'acides dicarboxyliques et d'aminoalcools, polyester-urée provenant d'aminoacides et de diesters de glycol ;
v) polymères polyhydroxylés sélectionnés dans le groupe consistant en polyvinylalcool, copolymères éthylène-alcool vinylique, totalement ou partiellement hydrolysés, et les polysaccharides allant jusqu'aux dextrines;
vi) polyvinylpyrrolidone, copolymères polyvinylpyrrolidone-acétate de vinyle, polyéthyloxazolines;
vii) polymères ionomères sélectionnés parmi polyacrylates et polyméthacrylates ;
d) polyesters obtenus à partir de monomères ou comonomères tels que définis ci-dessus aux points a) et b) améliorés par des agents d'extension de chaîne sélectionnés dans le groupe consistant en isocyanates, époxydes, phénylesters et carbonates aliphatiques;
e) polyesters obtenus à partir des monomères et comonomères définis aux points a) et b) ci-dessus, partiellement réticulés à l'aide d'acides polyfonctionnels sélectionnés dans le groupe consistant en acide trimellitique, acide pyrromellitique, polyisocyanates et polyépoxydes, les compositions obtenues à partir des constituants suivants étant exclues:
- amidon, polymère synthétique choisis parmi copolymères éthylène-alcool vinylique, polyalcool vinylique et polyalcool vinylique modifié et leurs mélanges, un second composant choisi parmi a) et b) polymères et plastifiants autres que sorbitol et esters acétiques de glycérol et choisis dans le groupe suivant:
- polyols contenant 1 à 20 motifs hydroxylés répétitifs, chaque motif comportant 2 à 6 atomes de carbone, à condition que lorsque le polyol est formé par un seul motif répétitif, il comporte au moins 4 atomes de carbone;
- éthers, thioéthers, esters organiques et inorganiques, acétals et dérivés aminés de polyols, formées de 1 à 20 motifs hydroxyles répétitifs comportant chacun 2 à 6 atomes de carbone.
- produits de la réactions de polyol ayant 1 à 20 motifs hydroxyles répétitifs comportant chacun 2 à 6 atomes de carbone avec des extenseurs de chaîne;
- produits d'oxydation de polyol ayant de I à 20 motifs hydroxylés répétitifs comportant chacun de 2 à 6 atomes de carbone et comportant au moins un groupe fonctionnel aldéhyde ou carboxylique ou leurs mélanges.

2. Une composition selon la revendication 1, dans laquelle le composant à base d'amidon et le composant polymère de synthèse sont présents selon un rapport pondéral de 1/9 à 9/1 et de préférence de 1/4 à 4/1.

3. Une composition selon une quelconque des revendications 1 ou 2 dans laquelle le composant de synthèse est un mélange d'un polymère a) et d'un copolymère b).

4. Une composition selon la revendication 1, dans laquelle le composant de synthèse est un polymère ou un copolymère d'un ε-hydroxyacide ou d'une lactone correspondante sélectionnée dans le groupe consistant en acides 6-hydroxycaproïque, 6-hydroxyoctanoïque et 3,7-diméthyl-6-hydroxyoctanoïque.

5. Une composition selon la revendication 4, dans laquelle le composant de synthèse et un copolymère d'un ε-hydroxyacide et d'un isocyanate sélectionné dans le groupe consistant en 4,4'-diphénylméthane-diisocyanate, toluilènediisocyanate, isophorondiisocyanate et hexaneméthylènediisocyanate.

6. Une composition selon les revendications 1 à 5, dans laquelle le composant de synthèse comprend du poly-ε-caprolactone ou un copolymère d'un poly-ε-caprolactone et d'un isocyanate aliphatique ou aromatique ou un mélange en dérivant ou un copolymère de poly-ε-caprolactone et de cellulose, cellulose modifiée, acétate de cellulose ou carboxyméthylcellulose.

7. Une composition selon la revendication 6, dans laquelle le composant de synthèse est une poly-ε-caprolactone présentant un poids moléculaire supérieur à 40 000.

8. Une composition selon la revendication 7, dans laquelle le composant de synthèse comprend un mélange de poly-ε-caprolactone et de copolymère d'ε-caprolactone avec un isocyanate aliphatique ou aromatique selon un rapport pondéral de 5/1 à 1/1.

9. Une composition selon la revendication 1, dans laquelle le composant de synthèse polymère comprend un mélange d'un composé A sélectionné parmi les polymères a) à e) et des mélanges en dérivant et d'un composant B comprenant un polymère dérivant de monomères éthyléniquement insaturés, ledit polymère présentant des unités répétitives comportant au moins un groupe polaire fonctionnel sélectionné dans le groupe consistant en hydroxyle, carboxyle, carboxyalkyle, alkylcarboxyle. pyrrolidyle et acétal. ladite composition comprenant un plastifiant choisi dans le groupe consistant en glycérol, glycérol mono- et di-acétates, sorbitol, éthylène et propylène glycols, éthylène ou propylène diglycols, éthylène ou propylène triglycols, polyéthylène glycol, polypropylène glycol, 1,2-propanediol, 1,3-propane diol.

10. Une composition selon la revendication 9, dans laquelle le composant B comprend un polymère sélectionné dans le groupe consistant en polyvinylalcool, copolymères d'éthylène-acide acrylique, éthylène-acétate de vinyle, éthylène-alcool vinylique, éthylène modifié-alcool vinylique, poly(alcool vinylique) modifié et les mélanges en dérivant.

11. Une composition selon la revendication 10, dans laquelle le composant 8 comprend un poly-éthylène-alcool vinylique obtenu par hydrolyse du poly-éthylèneacétate de vinyle correspondant présentant une teneur en éthylène inférieure à 44% en poids et un degré d'hydrolyse des groupes acétates de 50 à 100 %.

12. Une composition selon la revendication 9, dans laquelle les composants A et B sont présents selon un rapport pondéral de 1/6 à 6/1, de préférence de 1/4 à 4/1.

13. Une composition selon la revendication 9, comprenant :
- d'environ 20 à environ 60 % en poids du composant à base d'amidon ;
- d'environ 10 à environ 80 % en poids du composant A ;
- d'environ 0 à environ 45 % en poids du composant B,
les quantités en pour-cent étant exprimées par rapport à la somme de l'amidon et du total du composant de synthèse.

14. Une composition selon la revendication 13, comprenant d'environ 10 à environ 50 % en poids de composant A et d'environ 2 à environ 30 % en poids du composant B.

15. Une composition selon la revendication 9, comprenant :
- d'environ 5 à environ 60 % en poids du composant à base d'amidon,
- d'environ 40 à environ 80 % en poids du composant A;
- d'environ 0 à environ 35 % en poids du composant B.
les quantités en pour-cent étant exprimées par rapport à la somme de l'amidon et du total du composant de synthèse.

16. Une composition selon la revendication 15, comprenant d'environ 5 à environ 30 % en poids de composant B.

17. Une composition selon une quelconque des revendications 1 à 8. comprenant de 1 à 50 % en poids, par rapport au système, amidon/polymère de synthèse d'un plastifiant sélectionné dans le groupe consistant en glycérine. polyglycérol, glycérol éthoxylate, éthylène ou propyléneglycol, éthylène ou propylène diglycol, éthylène ou propylène triglycol, polyéthylène ou polypropylène glycol, 1,2-propanediol, 1,3-propanediol, 1,2-, 1,3-, 1,4-butanediol, 1,5-pentanediol, 1,5-hexanediol, 1,2,6-, 1,3,5-hexanetriol, néopentylglycol, triméthylolpropane, pentaérythritol, acétate de sorbitol, diacétate de sorbitol, monoéthoxylate de sorbitol, dipropoxylate de sorbitol, diéhoxylate de sorbitol, hexaéthoxylate de sorbitol, aminosorbitol, trihydroxyméthylaminométhane, glucose/PEG, le produit de la réaction de l'oxyde d'éthylène avec du glucose, triméthylolpropane monoéthoxylate, monoacétate de mannitol, monoéthoxylate de mannitol, butyl glucoside, monoéthoxylate de glucose, α-méthyl-glucoside, sel de sodium du carboxyméthylsorbitol, poly(monoéthoxylate de glycérol) et les mélanges en dérivant.

18. Une composition selon la revendication 17, dans laquelle le plastifiant est présent en une quantité de 1 à 50 % en poids par rapport au poids de la composition totale consistant en composant à base d'amidon et en composant(s) polymères.

19. Une composition selon la revendication 16, dans laquelle le plastifiant est sélectionné parmi la glycérine et l'éthoxylate de sorbitol, et les mélanges en dérivant.

20. Une composition selon la revendication 16, comprenant de l'urée en une quantité de 0,5 à 20 % en poids par rapport au poids de la composition totale.

21. Une composition selon la revendication 1 ou 8, comprenant jusqu'à 5 % en poids, par rapport à la composition globale, d'un polymère hydrophobe sélectionné dans le groupe consistant en polyéthylène, polypropylène et polystyrène.

22. Une composition selon une quelconque des revendications 1 à 8 dans laquelle le composant synthétique comprend un polymère ou copolymère selon a) à e) de la revendication 1 et un plastifiant choisi dans le groupe tel que défini dans la revendication 9.

23. Une composition selon la revendication 22 dans laquelle le plastifiant est utilisé en une quantité de 1 à 50% par rapport au poids de la composition.

24. Une composition selon la revendication 1 ou 8, présentant une teneur en eau de 1,5 à 5 % en poids par rapport à la composition totale.

25. Une composition selon une quelconque des revendications 1 à 24 comprenant un additif choisi dans le groupe consistant en acetate et nitrate de cellulose, cellulose régénérée, alkyl cellulose, carboxyméthylcellulose.

26. Une composition selon la revendication 1 ou 8, dans laquelle le composant à base d'amidon comprend plus de 78 % en poids d'amylopectine.

27. Une composition selon la revendication 1 ou 8, comprenant de 0,01 à 10 % en poids, par rapport au poids du composant à base d'amidon, d'un additif sélectionné dans le groupe consistant en acide borique, borate, acide métaborique, hydroxyde d'aluminium et sel de métal alcalin.

28. Une composition selon la revendication 23, dans laquelle le composant à base d'amidon présente une teneur en amylopectine supérieure à 70 % en poids.

29. Une composition selon la revendication 9 dans laquelle le composant A comprend un polymère obtenu par polymérisation d'un diol aliphatique avec un acide dicarboxylique.

30. Une composition selon la revendication 29 dans laquelle le polymère obtenu par polymérisation d'un diol aliphatique avec un acide dicarboxylique est choisi parmi les polyéthylène et polybutylène adipates et sébaçates.

31. Articles, et en particulier films, feuilles, fibres et filaments tels qu'ils sont susceptibles d'être obtenus par l'utilisation d'une composition polymère selon une quelconque revendications 1 à 8.

32. Un film ou feuille obtenues à partir d'une composition préparée à partir d'une masse fondue comprenant amidon, polycaprolactone et un plastifiant choisi parmi glycérine, sorbitol ou leurs mélanges.
